# EUROPÄISCHE PATENTSCHRIFT

(11) **EP 1 658 852 B1**
(45) Veröffentlichungstag und Bekanntmachung des Hinweises auf die Patenterteilung: **09.11.2016**
(21) Anmeldenummer: 06001911.4
(22) Anmeldetag: 21.04.1997
(51) Int. Cl.: A61K 31/663, A61K 9/28

(54) **Orale, pharmazeutische Zubereitung enthaltend Ibandronat**
Oral pharmaceutical preparation containing ibandronat
Préparation pharmaceutique orale contenant de l'ibandronate

(30) Priorität: 20.04.1996 DE 19615812
(43) Veröffentlichungstag der Anmeldung: 24.05.2006
(62) Teilanmeldung aus: 97918146.8
(73) Patentinhaber: Roche Diagnostics GmbH, 68305 Mannheim (DE)
(72) Erfinder: Gabel, Rolf-Dieter, 68723 Schwetzingen (DE); Moeckel, Joern, 69221 Dossenheim (DE); Woog, Heinrich, 69514 Laudenbach (DE)
(74) Vertreter: Vossius & Partner Patentanwälte Rechtsanwälte mbB

(56) Entgegenhaltungen:
- EP-A- 0 550 385
- EP-A- 0 566 535
- WO-A-94/12200
- WO-A-94/26310
- WO-A-95/08331
- DE-A- 3 623 397
- HERAUSGEGEBEN VON K. HARTKE ET AL: "DAB10-Kommentar, Wissenschaftliche Erläuterungen zum Deutschen Arzneibuch, 10. Ausgabe 1991, Teil 1: Allgemeiner Teil (Methoden und Reagenzien)" 1993, WISSENSCHAFTLICHE VERLAGSGESELLSCHAFT MBH , STUTTGART , XP002371970 V.5.4.

## Beschreibung

Die Erfindung betrifft pharmazeutische Zubereitungen von Ibandronat oder dessen physiologisch verträglichen Salze zur oralen Applikation, gemäß Anspruch 1.

Der Wirkstoff Ibandronasäure (1-Hydroxy-3-(N-methyl-N-pentyl)aminopropyl-1,1-diphosphonsäure) bzw. deren Salze (Ibandronate) gehören zur Klasse der Diphosphonsäuren, die vor allem von Interesse sind bei der Behandlung von Knochenerkrankungen und bestimmten Störungen des Calciumhaushalts, wie z.B. der Hyperkalzämie, Osteoporose, Tumorosteolyse oder Morbus Paget. Da zur Behandlung der erwähnten Erkrankungen diese Wirkstoffe häufig und über einen langen Zeitraum gegeben werden müssen, ist neben der intravenösen vor allem die orale Applikation anzustreben, da diese bei vielen Patienten auf eine höhere Akzeptanz trifft.

Die orale Behandlung wird jedoch grundsätzlich durch bekannte orale Verträglichkeitsprobleme der Diphosphonsäuren im allgemeinen erschwert. Es ist bekannt, daß Diphosphonsäuren bzw. deren physiologisch unbedenklichen Salze, und insbesondere Aminodiphosphonsäuren, zu Irritationen des oberen Gastrointestinaltrakts führen (Fleisch H, Bisphosphonates in Bone Disease, Herbert Fleisch, Bern 1993; S. 126 - 131). Dies trifft auch zu für Diphosphonate, die auch in relativ geringen Dosierungen von beispielsweise weniger als 50 mg pro Einzeldarreichungsform eingenommen werden. In WO 93/09785 wird darauf hingewiesen, daß beispielsweise der Wirkstoff Risedronat ([1-Hydroxy-2-(3-pyridinyl)-ethyliden]bis-phosphonat) zu Erosionen und Ulzerationen in den oberen Abschnitten des Verdauungstrakts führen kann. Verschiedene Literaturstellen weisen ferner auf gastrointestinale Unverträglichkeiten des Wirkstoffes Pamidronat (Dodwell D et al., Biochemical Effects, Antitumor Activity and Pharmacokinetics of Oral and Intravenous Pamidronate (APD) in the Treatment of Skeletal Breast Cancer, Br. J. Cancer 62 496 (1990)) und Tiludronat (Reginster J Y, Efficacy and Tolerability of a New Formulation of Oral Tiludronate (Tablet) in the Treatment of Paget's Disease of Bone, J. Bone Miner. Res. 9, 615 - 619 (1994)) hin. Es ist auch bekannt, daß außerdem Motalitätsstörungen beim Schlucken der Tabletten auftreten können bzw. die einzunehmenden Tabletten wegen besonderer anatomischer Gegenheiten im Ösophagus (Speiseröhre) hängen bleiben. Dabei können eine Odynophagie oder auch ösophageale Strikturen entstehen. Dies ist häufig der Fall bei älteren Patienten oder bei Patienten, die infolge ihrer Erkrankung die erforderlichen Tabletten vorwiegend im Liegen einnehmen müssen.

Dementsprechend wurden zur Lösung dieser Probleme in der Fachwelt die Forderung aufgestellt, oral verfügbare Darreichungsformen grundsätzlich mit einem magensaftresistenten Film zu überziehen, so daß der Wirkstoff erst nach der Passage durch den Magen freigesetzt wird, und somit Reizungen des Magens und der Speiseröhre vermieden werden. Beispielsweise werden in WO 95/08331 Darreichungsformen beschrieben, mit denen das Irritationspotential von Alendronat und anderen Diphosphonaten bei oraler Applikation verringert werden kann.

Aufgrund der beschriebenen oralen Unverträglichkeiten der Diphosphonate wurde bei einer Reihe dieser Wirkstoffe nach besser verträglichen Darreichungsformen gesucht. Dabei wurden insbesondere solche oralen Darreichungsformen entwickelt, die mit magensaftresistenten Überzüge überzogen sind. Derartige Überzüge sind das Mittel der Wahl um die oberen Abschnitte des Gastrointestinaltrakts, insbesondere die Speiseröhre oder den Magen, vor unverträglichen Wirkstoffen zu schützen. Diese magensaftresistenten Überzüge auf festen oralen Darreichungsformen lösen sich erst bei einem höheren pH-Wert ab ca. 5,5 auf, so daß im sauren Milieu des Magens, bei einem pH-Wert weit unter 5,5 kein Wirkstoff aus der Darreichungsform abgegeben wird und somit der Magen vor Irritationen durch den Wirkstoff geschützt ist. Da kein Wirkstoff im Magen freigesetzt wird, kann gleichzeitig verhindert werden, daß es durch Reflux wirkstoffhaltigen Mageninhalts zu Ösophagitiden oder anderen Irritationen der Speiseröhre kommt. Folgerichtig wird in DE 59 005 517 (EP 0 421 921) eine magensaftresistente orale Darreichungsform für Pamidronat beschrieben, die geeignet ist, das Risiko von Magenulzera zu reduzieren. WO 93/09785 schildert ferner eine solche Darreichungsform für Risedronat, WO 95/08331 für Alendronat und andere Disphosphonate.

Weitere Hintergrundinformationen finden sich in WO 94/12200 und EP 0 566 535.
Den möglichen Vorteilen der magensaftresistenten Darreichungsformen stehen eine Reihe von Nachteilen gegenüber. So kann bei diesen Arzneiformen die Resorption gegenüber den pH-unabhängig schnell freisetzenden Formen verringert sein oder die Resorption ist im Vergleich zu konventionellen Formen von wesentlich höherer Variabilität und damit die Therapiesicherheit beeinträchtigt oder in Frage gestellt. Daher besteht Bedarf an einer alternativen Darreichungsform für derartige Wirkstoffe, um die Nachteile der magensaftresistenten Formen zu umgehen, aber dennoch ausreichenden Schutz vor diesen auf die Magenschleimhaut aggressiv wirkenden Aminobisphosphonaten bieten zu können.
Überraschenderweise wurde nun gefunden, daß im Falle des Wirkstoffes Ibandronat bereits dann eine verbesserte orale Verträglichkeit erzielt wird, wenn perorale Darreichungsformen derart mit einer Hilfsstoffschicht oder einem Film überzogen werden, daß sich der Wirkstoff in kurzer Zeit auflöst und entsprechend hohe lokale Konzentrationen des Wirkstoffes im Magen erzielt werden. Der sich beim Kontakt mit den Verdauungssäften auflösende Film ist vorzugsweise ein Überzug, der sich unabhängig vom pH-Wert auflöst. Das Aufbringen dieser Hilfsstoffschicht kann mit pharmazeutisch-technologisch üblichen Verfahren erfolgen. Obwohl diese Schicht nicht die Auflösung des lbandronats im Magen verhindert, wurden in klinischen Studien überraschenderweise auch mit hochdosiertem Ibandronat keine signifikanten Nebenwirkungen beobachtet. Trotz der Tatsache, daß die Darreichungsformen mit einem den Wirkstoff schnell freisetzenden Film überzogen sind, kommt es beim Schlucken der Tabletten nicht zu Reizungen in der Speiseröhre und es treten keine Ösophagitiden auf Dies ist insbesondere von Vorteil, da die erfindungsgemäßen Darreichungsformen auch von im Bett liegenden Patienten gut vertragen werden.
Erfindungsgemäß werden Filmtabletten von Ibandronat zur Verfügung gestellt, die aus einem wirkstoffhaltigen Kern bestehen, der mit einer wirkstoff-freien Hilfsstoffschicht überzogen ist, die bei Kontakt mit Verdauungssäften entweder unabhängig vom pH-Wert in Lösung geht oder sich von der festen oralen Zubereitung ablöst. Hierdurch wird sichergestellt, daß die Arzneiform relativ schnell zerfällt und der Wirkstoff in kurzer Zeit freigesetzt wird, wodurch hohe lokale Konzentrationen des Wirkstoffes erzielt werden. Der Überzug kann durch Verfahren wie Filmcoating, Preßcoating, Dragierung, Verkapselung oder Mikroverkapselung aufgebracht werden. Die Freisetzung des Wirkstoffs aus den entsprechend überzogenen
   Filmtabletten
erfolgt im Vergleich zu den magensaftresistenten Darreichungsformen beschleunigt. Erfindungsgemäß werden mindestens 30 % der enthaltenen Ibandronat-Dosis, bevorzugt aber mindestens 75 % und insbesondere mindestens etwa 85 % innerhalb der physiologischen pH-Bandbreite unabhängig vom pH-Wert schnell freigesetzt. Die Zeit, innerhalb der diese Prozentsätze in der Freisetzung erreicht werden, beträgt vorzugsweise weniger als etwa 2 Stunden, vorzugsweise weniger als 1 Stunde, besonders bevorzugt etwa 1 - 30 Minuten. Besonders bevorzugt beträgt die Freisetzung etwa 80 - 90 % innerhalb einer Zeit von bis zu 15 Minuten. Die Freisetzung des Wirkstoffes wird zweckmäßigerweise im Rahmen eines in vitro Versuches nach allgemein bekannten standardisierten Verfahren bestimmt.
Die schnelle Freisetzung des Wirkstoffes führt überraschenderweise trotz der dadurch entstehenden hohen lokalen Konzentration des Wirkstoffes (d.h. trotz hohem Wirkstoffgradienten) im Magen nicht zu den üblicherweise für Disphosphonsäure bekannten unerwünschten Nebenwirkungen wie oben beschrieben. Vielmehr wurde gefunden, daß trotz der raschen Freisetzung des Wirkstoffes das Problem des Auftretens gastrointestinaler Störungen völlig überraschend vermieden wird. Außerdem wurde beobachtet, daß bei den mit derartigen schnellsetzenden Darreichungsformen von Ibandronat behandelten Patienten deutlich weniger Falle an Übelkeit, Erbrechen, Schmerzen oder Diarrhöe zu beobachten waren, die sonst bei der Verabreichung von Aminobisphosphonaten beobachtet wurden.
Die im Sinne der vorliegenden Erfindung den Wirkstoff schnell freisetzenden Überzüge können auf Tabletten, aufgetragen werden. Diese Tabletten bestehen aus Mischungen von Wirkstoffen mit pharmazeutischen Hilfsstoffen oder aus reinen Wirkstoffen. Der Überzug kann mit verschiedenen pharmazeutisch üblichen Verfahren erfolgen. Geeignete Verfahren nutzen z.B. Dragieranlagen, Anlagen für das Filmcoating, Tablettenpressen für das Preßcoating, Kapselmaschinen oder Anlagen für das Mikroverkapseln, wie z.B. Einrichtungen zur Herstellung von Sprüherstarrungen und Sprüheinbettungen, Einrichtungen zur Herstellung von einfachen oder komplexen Koazervaten.
Als Filmbildner im Sinne der Erfindung kommen pharmazeutisch übliche oder physiologisch unbedenkliche Polymere in Frage. Filmbildner im Sinne der Erfindung stammen z.B. aus den Gruppen der Cellulosederivate, Dextrine, Stärken und Stärkederivate, Polymere auf Basis sonstiger Kohlehydrate und deren Derivate, natürliche Gummen wie Gummi Arabikum, Xanthane, Alginate, Polyacrylsäure, Polyvinylalkohol, Polyvinylacetat, Polyvinylpyrrolidon, Polymethacrylate und deren Derivate (Eudragit ^{®}), Chitosan und dessen Derivate, Schellack und dessen Derivate sind ebenfalls als Filmbildner offenbart. Neben diesen Filmbildnern können auch Substanzen aus der Stoffklasse der Wachse und Fette zur Herstellung von Überzügen dienen.
Im Falle der Cellulosederivate kommen bevorzugt die löslichen Alkyl- öder Hydroxy-alkylcellulose-Derivate in Frage, wie z.B. Methylcellulose, Hydroxymethylcellulose, Hydroxyethylcellulose, Hydroxypropylcellulose, Methylhydroxyethylcellulose, Methyl-hydroxypropylcellulose oder Natriumcarboxymethylcellulose. In einer bevorzugten Ausführungsvariante der Erfindung kommt Methylhydroxypropylcellulose zum Einsatz. Als geeignete Filmbildner auf Cellulosebasis können die üblichen für pharmazeutisch Zwecke geeigneten Cellulosederivate mit unterschiedlichem Substitutionsgrad und/oder unterschiedlichem Molekulargewicht, entsprechend einem unterschiedlichen Viskositätsgrad der wäßrigen Lösung, eingesetzt werden. Auch unlösliche Cellulose-derivate wie z.B. Ethylcellulose können eingesetzt werden.
Im Falle der Polymethacrylate kommen kationische Copolymerisate von Dimethylaminoethylmethacrylat mit neutralen Methacrysäureestem (Eudragit^{®} E), Copolymerisate aus Acryl- und Methacrylsäureestern mit einem geringen Gehalt quartärer Ammoniumgruppen (in "Ammonio Methacrylate Copolymer Type A bzw. Type B" USP/NF beschrieben, Eudragit^{®} RL bzw. RS) und Copolymerisate von Ethylacrylat und Methylmethacrylat mit neutralem Charakter (in Form einer wäßrigen Dispersion, in "Polyacrylate Dispersion 30 Per Cent" Ph. Eur. beschrieben, Eudragit^{®} NE 30 D) in Frage.
Auch der Einsatz von Filmbildnern, die üblicherweise zur Herstellung magensaftresistenter Filme genutzt werden ist denkbar, solange z.B. durch geringe Schichtdicke des Auftrags oder andere Maßnahmen wie extrem hoher Anteil an Porenbildner o.a. gewährleistet ist, daß der Wirkstoff aus der entsprechend überzogenen Darreichungsform wie oben beschrieben pH-unabhängig schnell freigesetzt wird. Als solche Filme können zum Einsatz kommen: Anionische Copolymerisate aus Methacrylsäure und Methylmethacrylat (in "Methacrylic Acid Copolymer, Type C" USP/NF beschrieben, Eudragit^{®} L bzw. S oder in Form der wäßrigen Dispersion Eudragit^{®} L 30 D), saure Cellulosederivate wie Celluloseacetatphthalat, Celluloseacetattrimellitat und Methyl-hydroxypropylcellulosephthalat, Polyvinylacctatphthalat u.a..
Alle Filmbildner können prinzipiell sowohl einzeln, als auch in Gemischen von zwei oder mehreren Filmbildnem verwendet werden.
Die Filme können bei Bedarf zusätzliche Hilfsstoffe wie Weichmacher, Porenbildner, Füllstoffe, Farbstoffe, Pigmente, Antischaummittel, Antiklebemittel u.a. enthalten.
Für das Preßcoating kommen Polymere und die eventuell zusätzlich benötigten Hilfsstoffe in Frage, wie sie im vorangegangenen Abschnitt beschrieben wurden, soweit sie sich mit der Preßcoating-Technologie verarbeiten lassen. Zusätzlich sind alle pharmazeutisch üblichen er physiologisch verträglichen Hilfsstoffe zu verwenden, die geeignet sind, beim Preßcoating eine geschlossene Hülle auf der zu umhüllenden Arzneiform zu erzeugen. Dazu gehören insbesondere Hilfsstoffe, wie sie für die konventionelle Tablettierung üblich sind, im speziellen Füllstoffe aus der Gruppe der Kohlehydrate wie Lactose, Saccharose, Glukose und andere Zucker, Mikrokristalline Cellulose, Stärken und Stärkederivate, Zuckeralkohole wie z.B. Mannit, Sorbit, Xylit, anorganische Füllstoffe wie beispielsweise Phosphate, Carbonate. Neben den Füllstoffen können erfindungsgemäß andere Hilfsstoffe enthalten sein, wie sie zur Herstellung konventioneller Tabletten nötig sind, wie Bindemittel, Sprengmittel, Fließmittel. Formentrennmittel, Geschmackskorrigentien, Pigmente und Farbstoffe.
Für die Dragierung können prinzipiell alle Hilfsstoffe eingesetzt werden, die bereits zuvor beschrieben wurden. Zusätzlich können hier spezielle Hilfsstoffe für die Dragierung eingesetzt werden wie Palatinit, Bentonit, Calciumsulfat als Füllstoffe, Polyethylenglykol oder Polyethylenglykolfettsäureester als Trennmittel, kolloidale Kieselsäure als Trockenmittel und Strukturbildner, Magnesiumoxid als Einstreupulver, pharmazeutisch übliche oder physiologisch unbedenkliche Fette und Wachse als Glanzrnittel.
Als Kapseln kommen alle pharmazeutisch üblichen Kapseln in Frage wie z.B. Gelatinehartkapseln, Weichgelatinekapseln, Stärkekapseln. Die Kapseln können mit Pulvern, Granulaten, Pellets oder Tabletten gefüllt sein. Generell ist die Abfüllung aller pharmazeutisch üblichen festen, flüssigen und halbfesten Zubereitungen in
   Kapseln möglich.
Für die Mikroverkapselung des Wirkstoffs oder von Wirkstoffzubereitungen können
   alle polymeren Filmbildner eingesetzt werden, die zuvor genannt wurden. Die Polymere können dabei sowohl einzeln, als auch in Gemischen aus mehreren Polymeren, wenn nötig auch zusammen mit anderen Hilfsstoffen eingesetzt werden.
Nachfolgend soll die Erfindung durch Ausführungsbeispiele verdeutlicht werden, ohne sie darauf einzuschränken.
Der Wirkstoff Ibandronat wird beispielsweise in Mengen von 0,1 - 100 mg pro Einzeldosierungseinheit eingesetzt. Bevorzugt beträgt die Dosierung mindestens etwa 1 mg. 5 mg, 10 mg oder 20 mg als untere Grenze film die Wirkstoffmenge in der Einzeldarreichungsform. Die obere Grenze liegt bei etwa 250 mg, insbesondere 100 mg bzw. 50 mg.

### Beispiel 1

Ibandronathaltiger Kern:

| | | | |
|---|---|---|---|
| Ibandronat-Dosis [mg] in 200 mg-Tablettenkern: | 10,0 | 20,0 | 50,0 |

Ibandronatfreie Hülle [mg]:

| | | | |
|---|---|---|---|
| Methylhydroxypropylcellulose | 5,1425 | 5,1425 | 5,1425 |
| Titandioxid | 2,4650 | 2,4650 | 2,4650 |
| Macrogol | 1,5000 | 1,5000 | 1,5000 |
| Talkum | 0,8925 | 0,8925 | 0,8925 |
| Filmauftrag gesamt | 10,0000 | 10,0000 | 10,0000 |

Das Filmcoating erfolgt in üblichen Apparaten. Coatingbedingungen: Tabletten-Einlage: 140 kg; Zulufttemperatur: 60 °C.

### Bespiel 2:

Ibandronathaltiger Kern:

| | | | |
|---|---|---|---|
| Ibandronat-Dosis in 100 mg-Tablettenkern [mg]: | 0,1 | 2,5 | 5,0 |

Ibandronatfreie Hülle [mg]:

| | | | |
|---|---|---|---|
| Methylhydroxypropylcelulose | 2,057 | 2,057 | 2,057 |
| Titandioxid | 0,986 | 0,986 | 0,986 |
| Macrogol | 0,600 | 0,600 | 0,600 |
| Talkum | 0,357 | 0,357 | 0,357 |
| Filmauftrag gesamt | 4,000 | 4,000 | 4,000 |

Filmcoating in konventionellem 2501-Dragierkessel. Coatingbedingungen: Tabletten-Einlage 144 kg.

### Beispiel 3:

Ibandronathaltiger Kern:

| | | | |
|---|---|---|---|
| Ibandronat-Dosis [mg] in 200 mg-Tablettenkern: | 10,0 | 20,0 | 50,0 |

Ibandronatfreie Hülle [mg]:

| | | | |
|---|---|---|---|
| Talkum | 2,00 | 2,00 | 2,00 |
| Lactose | 1,40 | 1,40 | 1,40 |
| Methylhydroxypropylcellulose | 0,80 | 0,80 | 0,80 |
| Titandioxid | 0,80 | 0,80 | 0,80 |
| Macrogol | 0,40 | 0,40 | 0,40 |
| Ethylmethylmethacrylat Copolymer * | 0,04 | 0,04 | 0,04 |
| Polysorbat | 0,04 | 0,04 | 0,04 |
| Filmauftrag gesamt | 5,48 | 5,48 | 5,48 |

| | | | |
|---|---|---|---|
| * eingesetzt in Form von Eudragit™ NE 30 D als wäßrige Dispersion | | | |

Filmcoating in konventionellem 151-Dragierkessel.
Coatingbedingungen: Tabletten-Einlage: 13,0 kg.

### Beispiel 4:

Ibandronathaltiger Kern:

| | |
|---|---|
| Ibandronat-Dosis in 74 mg-Tablettenkern [mg]: | 20 |

Ibandronatfreie Hülle [mg]:

| | |
|---|---|
| Methylhydroxypropylcellulosephthalat | 4,580 |
| Triacetin | 1,374 |
| Polysorbat | 0,046 |
| Filmauftrag gesamt | 6,000 |

Filmcoating in konventionellem 51-Dragierkessel.
Coatingbedingungen: Tabletten-Einlage: 0,25kg.

### Beispiel 5:

Ibandronathaltiger Kern:

| | |
|---|---|
| Ibandronat-Dosis [mg] in 86 mg-Tablettenkern: | 10 |

Ibandronatfreie Hülle [mg]:

| | |
|---|---|
| Saccharose | 37,844 |
| Weisser Ton | 8,138 |
| Talkum | 1,000 |
| Macrogol | 2,848 |
| Glucosesirup | 2,035 |
| Titandioxid | 1,628 |
| Polyvidon | 0,407 |
| Montanglycolwachs | 0,100 |
| Dragierauftrag gesamt | 54,000 |

Dragierung in konventionellem 151-Dragierkessel mit Tauchrohr.

### Bespiel 6:

Ibandronathaltiger Kern:
   Ibandronat-Dosis [mg] in 400 mg-Granulat: 20 mg und 50 mg
   Ibandronatfreie Hülle [mg]: Hartgelatinesteckkapsel Größe 0, weiß opak.
   Die Verkapselung der Arzneimittelmasse erfolgt auf einer Kapselmaschine vom Typ Harro-Höfliger.

### Beispiel 7:

Ibandronat in Manteltablette
Ibandronathaltiger Kern:

| | |
|---|---|
| Endgewicht: | 86 mg |
| Kernformat: | 7 mm Durchmesser, plan, facettiert |
| Ibandronat-Dosis in Tablettenkern: | 10 mg |

Ibandronatfreie Hülle:

| | |
|---|---|
| Lactose | 270 mg |
| Mikrokristalline Cellulose: | 90 mg |
| Preßwerkzeug: | 12 mm Durchmesser |
| Endgewicht Manteltablette: | 446 mg |

Das Verpressen erfolgt mitttels Handpresse nach konventionellen Verfahren.

## Patentansprüche

1. Filmtablette, enthaltend Ibandronat oder ein physiologisch verträgliches Salz davon als Wirkstoff, **dadurch gekennzeichnet, dass** die Filmtablette aus einem wirkstoffhaltigen inneren Teil besteht, der von einer wirkstoff-freien Hülle derart umgeben ist, dass mindestens 75% des Wirkstoffes innerhalb einer halben Stunde bei Kontakt im wässrigen Medium bei pH 1 bis 7,4 freigesetzt wird.

2. Filmtablette nach Anspruch 1, **dadurch gekennzeichnet, dass** die wirkstoff-freie Hülle mindestens eines der folgenden Cellulosederivate enthält: Methylcellulose, Hydroxymethylcellulose, Hydroxyethylcellulose, Hydroxypropylcellulose, Methylhydroxyethylcellulose, Methylhydroxypropylcellulose, Natriumcarboxymethylcellulose, Ethylcellulose.

3. Filmtablette nach Anspruch 1 oder 2, **dadurch gekennzeichnet, dass** die wirkstoff-freie Hülle mindestens einen Weichmacher, Porenbildner, Füllstoff, Farbstoff, Pigment, Antischaummittel und/oder Antiklebemittel enthält.

4. Filmtablette nach einem der Ansprüche 1 bis 3 zur Verwendung bei der Behandlung von Calciumstoffwechselerkrankungen unter Vermeidung von Ösophagitiden.

## Claims

1. Film tablet containing ibandronate or a physiologically acceptable salt thereof as active ingredient, **characterised in that** the film tablet consists of a core containing an active ingredient which is coated by an active ingredient-free film in such a way that at least 75% of the active ingredient is released within thirty minutes upon contact in aqueous medium at pH 1 to 7.4.

2. Film tablet according to claim 1, **characterised in that** the active ingredient-free film contains at least one of the following cellulose derivatives: methylcellulose, hydroxymethylcellulose, hydroxyethylcellulose, hydroxypropylcellulose, methylhydroxyethylcellulose, methylhydroxypropylcellulose, sodiumcarboxymethylcellulose, ethylcellulose.

3. Film tablet according to claim 1 or 2, **characterised in that** the ingredient-free film contains at least one plasticizer, pore-forming agent, filling agent, dye, pigment, anti-foaming agent and/or anti-adhesion agent.

4. Film tablet according to any one of claims 1 to 3 for use in the treatment of calcium metabolism diseases while avoiding esophagitis.

## Revendications

1. Comprimé pelliculé contenant de l'ibandronate ou un sel physiologiquement acceptable de celui-ci comme principe actif, **caractérisé en ce que** le comprimé pelliculé consiste en une partie intérieure contenant le principe actif, qui est entourée d'une enveloppe sans principe actif, de telle sorte qu'au moins 75 % du principe actif soient libérés en une demi-heure par contact dans un milieu aqueux à pH 1 à 7,4.

2. Comprimé pelliculé selon la revendication 1, **caractérisé en ce que** l'enveloppe sans principe actif contient au moins l'un des dérivés de cellulose suivants : méthylcellulose, hydroxyméthylcellulose, hydroxyéthylcellulose, hydroxypropylcellulose, méthylhydroxyéthylcellulose, méthylhydroxypropylcellulose, carboxyméthyl-cellulose de sodium, éthylcellulose.

3. Comprimé pelliculé selon la revendication 1 ou 2, **caractérisé en ce que** l'enveloppe sans principe actif contient au moins un plastifiant, un agent porogène, un agent de charge, un colorant, un pigment, un agent anti-mousse et/ou un agent anti-agglomérant.

4. Comprimé pelliculé selon l'une des revendications 1 à 3, pour son utilisation dans le traitement des maladies du métabolisme du calcium en évitant les oesophagites.
